# EUROPEAN PATENT SPECIFICATION

(11) **EP 3 810 081 B1**
(45) Date of publication and mention of the grant of the patent: **19.11.2025**
(21) Application number: 18923739.9
(22) Date of filing: 22.06.2018
(51) Int. Cl.: A61K 8/64, A61Q 5/00, A61Q 5/02, A61Q 5/12

(54) **COSMETIC COMPOSITION FOR TREATING KERATIN FIBERS**
KOSMETISCHE ZUSAMMENSETZUNG ZUR BEHANDLUNG VON KERATINFASERN
COMPOSITION COSMÉTIQUE POUR LE TRAITEMENT DE FIBRES DE KÉRATINE

(43) Date of publication of application: 28.04.2021
(73) Proprietor: Natura Cosméticos S.A., 05106-000 São Paulo SP (BR)
(72) Inventor: HANNEL BUELONI, Renata, 05106-000 São Paulo - SP (BR); ROESLER, Roberta, 05106-000 São Paulo - SP (BR); ANDRADE ARCURI, Helen, 05106-000 São Paulo - SP (BR); DE ANDRADE FREGONESI, Adriana, 05106-000 São Paulo - SP (BR); SCANAVEZ, Carla, 05106-000 São Paulo - SP (BR); ARRUDA COSTA, Andrea, 05106-000 São Paulo - SP (BR)
(74) Representative: Simmons & Simmons
(86) International application number: PCT/BR2018/050206
(87) International publication number: WO 2019/241859

(56) References cited:
- WO-A2-2004/073644
- DE-A1- 102014 225 582
- KR-B1- 101 687 471
- US-A1- 2005 019 297
- US-A1- 2007 196 429
- US-A1- 2009 123 967
- US-A1- 2015 079 012
- US-A1- 2016 280 960
- DATABASE GNPD [online] MINTEL; 1 December 2016 (2016-12-01), ANONYMOUS: "Lifting Stick", XP055891750, retrieved from https://www.gnpd.com/sinatra/recordpage/4461129/ Database accession no. 4461129

## Description

### SUMMARY OF THE INVENTION

The object of the present invention is a cosmetic composition for treating keratin fibers comprising a special selection of an amount of ADF-4 recombinant silk protein, such a selection providing unexpected effects to said composition, particularly desired deep and superficial treatment of keratin fibers.

Further, the present invention relates to a method for treating keratin fibers and the use of ADF-4 recombinant silk protein, in a selected amount, to produce a cosmetic composition with improved unexpected effects.

### BACKGROUND OF THE INVENTION

The hair fiber is a natural composite with unique physical and chemical properties. The hair shaft is made up of three main layers: cuticle, cortex and medulla.

The cuticle is the outer layer, and it is made up of many layers of translucent overlapping scales that protect the inner parts of the hair shaft. These scales should normally be smooth and lie flat or closed from root to point. This gives shiny and health hair. Hair in good condition is referred to as nonporous. Hair that has opened cuticles or that is dull or lacks shine is referred to as porous, dry or damaged hair (Flynn et al., Hair Loss Matters: A Handbook for Hairdressers and Barbers, AuthorHouse UK, 2016).

The cortex lies beneath the cuticle. The cortex is the main body of the hair, giving it bulk, strength, and elasticity. Hair is made up of amino acids and peptide chains that originate in the hair follicle. The amino acids and polypeptide chains are held together by permanent and temporary bonds in the cortex layer of the hair. Melanin, the natural color pigment, is also found in the cortex. Therefore, all chemical processes, such as coloring, bleaching, perming and chemical straightening, take place in the cortex. These processes must pass through the cuticle in order to do so (Flynn et al., 2016).

The medulla is the central layer of the hair. It has no function with regard the hairdressing processes, hair care or hair loss (Flynn et al., 2016).

The external layers of the hair are composed of keratin, a fibrous protein that is composed of several units of the amino acid cysteine. The amino acid cysteine that is present all over the extension of the keratin polymer contains sulfur. Through oxidation, two molecules of cysteine can provide strong disulfide links and, thus, bind adjacent keratin polymers. Such cysteine links contribute to the strength of the hair and are responsible for its durability and resistance.

Despite its robust nature, the hair shaft is not indestructible and lesions on/in the hair are common. Daily, the hair is exposed to several kinds of stress. Some of them are induced by environmental factors, such as UVA and UVB radiation, or by mechanical treatments using combs, brushes, heat drying devices, among others. Also, there is the stress caused by chemical processes in the form of dyes, permanents, smoothing and bleaching. These processes not only destroy the cysteine links (disulfide bridges), but also cause the loss of proteins (protein mass), loss of hydration, premature aging, hardness, fragility, rupture of fibers, and the like (as per "Chemical and Physical Behavior of Human Hair", Fourth Edition, Clarence R. Robbins).

In this sense, elasticity describes the ability of the hair shaft to stretch when wet and return to its natural state. Hair that can stretch up to 30 percent more than its original length and return to normal when released is referred to as healthy or nonporous hair. Hair that stretches and partially returns is considered porous or damaged. When hair is stretched and does not return but breaks, is referred to as hair breakage. The hair bulk comes from the amount of polypeptide chains in the cortex. Its strength comes from the natural state of the cortex. Too many chemical treatments will weaken the chains and bonds in the cortex and therefore result in poor elasticity, which indicates weak, damaged or hair breakage.

Compositions for the care of keratin fibers are known, but the majority is not able to provide simultaneous superficial (cuticle) and deep (cortex bulk) treatments. For example, KR 101 687 471 B1 discloses the treatment of hair with cosmetic products comprising peptides of plant origin. In particular, the mass and structure of the cortex of the hair fibres is increased by the treatment.

There is a need for a composition that achieves simultaneous superficial and deep repair of damage to keratinous substrates.

Biomimetics or biomimicry is the imitation of the models, systems, and elements of nature for the purpose of solving complex problems. Biomimetics has given rise to new technologies inspired by biological solutions at macro and nanoscales. Humans have looked at nature for answers to problems throughout our existence.

Throughout history, the innovation in materials stimulates technological evolutions. Many research institutes and R&D centers around the world are inspired by elements of the nature. In particular, there are extensive studies of the genetic design found in nature.

In this sense, spider silks caught attention because they are protein polymers that display extraordinary physical properties. Most silks have exceptional mechanical properties. They exhibit a unique combination of high tensile strength and extensibility (ductility). This enables a silk fiber to absorb a lot of energy before breaking (toughness, the area under a stress-strain curve).

Spider silk proteins are modular in nature because of their highly repetitive consensus sequences containing specific structural motifs. The different combination of these conserved motifs directly affects the mechanical properties of the fibers. Genetic manipulations allow for the design and creation of a library of synthetic repetitive silk-like genes with given protein sequences that target specific mechanical properties in the resulting fibers. Artificial combinations of different structural motifs (modules) native to spider silk proteins, such as the glycine-proline-glycine elastic motif (GPGXX/GPGGX), and the strength motifs composed of glycine and alanine (GA)n or two glycines and a third variable amino acid (GGX)n, may help to elucidate their individual functional roles and mode of interaction in the supramolecular fiber network. Ultimately, results of such fundamental structure- function relationships should facilitate the production of custom designed fibrous materials made from shorter silk-analog versions.

Each spider and each type of silk has a set of mechanical properties optimized for their biological function.

Spider silk proteins have been investigated mainly regarding their contribution to mechanical properties of the silk thread. Among the different types of spider silks, draglines are the most intensely studied.

Dragline silks are generally composed of two major proteins (ADF-3 and ADF-4) whose primary structures share a common repetitive architecture.

While some structural aspects of spider silk proteins have been unraveled, still little is known about the contribution of individual silk proteins and their primary structure elements to the assembly process. Comparative studies of the two major dragline silk proteins of the garden spider *Araneus diademalus,* ADF-3 and ADF-4, revealed that, although their amino acid sequences are rather similar, they display remarkably different solubility and assembly characteristics: While ADF-3 is soluble even at high concentrations, ADF-4 is virtually insoluble and self-assembles into filamentous structures under specific conditions (US 8,034,897, from Amsilk GMBH).

The use of natural proteins is well known and has been widely practiced in the cosmetics field, in particular the use of spider silk proteins.

It has been a goal to provide recombinant spider silk proteins having enhanced characteristics as, in particular, improved capability of being expressed in high yield and improved strength and flexibility, *i.e.* better quality.

The patent US 8,034,897 of Amsilk GmbH, discloses recombinant spider silk proteins, nucleic acids, coding for these recombinant spider silk proteins, as well as hosts suitable for expressing those nucleic acids. The nucleic acid sequence provided is ADF-3 and/or ADF-4, or a variant thereof. Two different kinds of ADF-3 and ADF-4 coding sequences and proteins are contemplated in US 8,034,897.

It is known that the use of spider silk recombinant proteins allows producing cosmetic or dermatological compositions with better properties, in particular good moisturizing and plasticizing effect. Spider silk recombinant proteins moreover behave as good water-resistant film-forming agents, and especially have a low surface density.

There are products in the market that use spider silk recombinant proteins in hair care cosmetic products, but these products can only treat the layers of the cuticle (around 2 µm) and do not reach the cortex of the hair fiber.

The majority of the cosmetic compositions available in the market comprise water, forming oil-in-water structures, what makes it difficult to obtain deep care, resulting in only superficial care of the keratinous substrates.

Compositions that provide deep care of keratinous substrates are even more difficult to achieve, especially for keratin fibers, including hair, eyebrows, eyelashes, mustache, beard, among others.

WO2015117888 from AMSILK GMBH deals with the application of a spider silk recombinant protein in the form of film coating with the property of delivery system of active agents with general description of application in skin and hair, focusing on pharmaceutical application and medicines.

US2002064539 from L'oreal and AMSILK deals with a cosmetic or dermatological composition contacting at least one natural or recombinant spider silk protein, deals with application in hair through the mechanism of surface film formation.

US2009123967 also from AMSILK describes the biotechnological production of various recombinant spider silk proteins, including ADF-4, and their possible application in skin and hair. It is a patent for the process of biotechnological production of proteins and not for application of active ingredient in hair.

Therefore, the prior art extensively directs the one skilled in the art to use recombinant spider silk proteins to prepare cosmetic compositions for superficial treatment or styling of keratin fibers due to the well know mechanism of surface film formation.

### BRIEF DESCRIPTION OF THE INVENTION

It has now been discovered that a specific spider silk recombinant protein (ADF-4 recombinant) gives unforeseen properties to cosmetic compositions, when used in a specific selected range percentage amount, in particular allowing a combined improved and unexpected action of both deep (cortex) and superficial (cuticle) fiber repair.

Therefore, the present invention relates to a cosmetic composition for treating keratin fibers comprising a special selection of a specific amount of ADF-4 recombinant silk protein, as well as a method for treating keratin fibers and the use of ADF-4 recombinant silk protein to produce a cosmetic composition.

Only classical film forming mechanisms are referred to in the prior art. No document teaches the interaction with the fiber as disclosed in the present invention. Unexpectedly, the methodologies described here demonstrate the unprecedented interaction between the biotechnological recombinant protein of the spider and the fiber that allows the double efficacy at the same time, obtained by the selected range amount.

### BRIEF DESCRIPTION OF THE FIGURES

The invention is further illustrated by the accompanying figures, wherein:

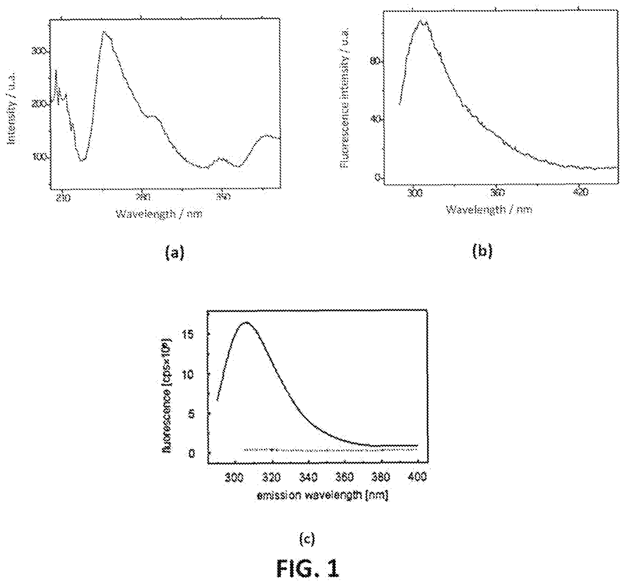

Figure 1 shows emissions of fluorescence confirming the existence of the recombinant protein in the sample submitted for analysis, wherein (a) is excitation spectrum of 200 to 500 nm, (b) is excitation emission spectrum at 280 nm, and (c) is emission spectrum from the literature, with emission at 280 nm (full line) and at 295 nm (dotted line).

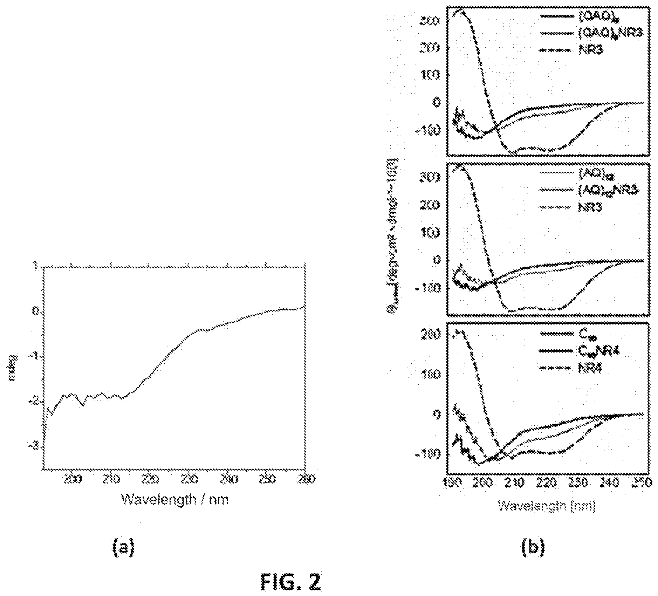

Figure 2 illustrates circular dichroism spectrum obtained experimentally (a) and literature spectra (b), where full-line spectra are of proteins containing only repeating units; dashed dotted spectra are of proteins composed exclusively of non-repetitive domains and gray line spectra are of recombinant proteins containing both portions.

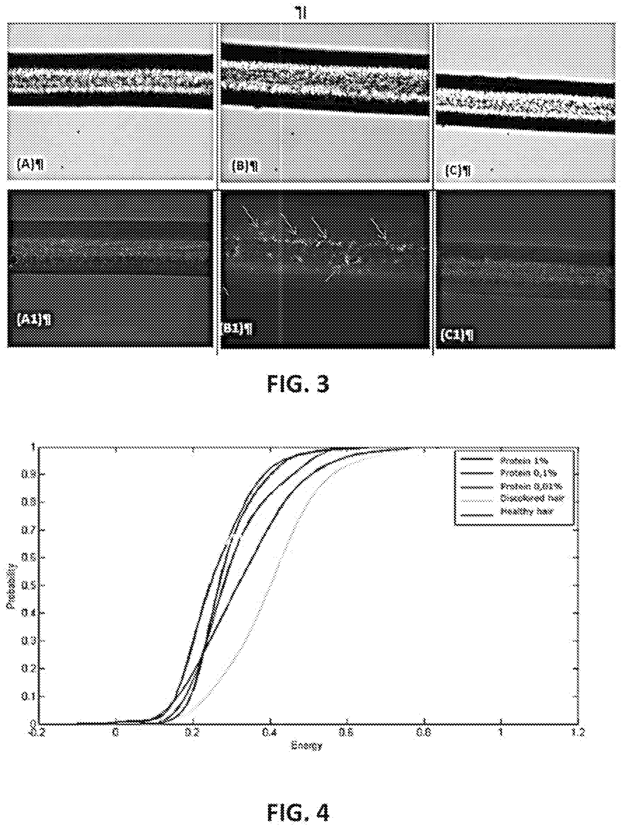

Figure 3 shows a comparison between (A) control hair which is a healthy hair without damage; (B) discolored hair; and (C) discolored hair treated with the protein ADF-4. The surface of the hair shaft was evaluated by analysis of digital images of texture: (A1) No cuticle damage = high texture uniformity; (B1) After cuticle damage = texture uniformity decreases; (C1) After repairing treatment with ADF-4 = recovery of the texture uniformity. Therefore, it is possible to observe the hair recovery texture uniformity after repairing treatment with ADF-4 protein (C1).
Figure 4 illustrates a graphic evaluating the re-texturization of the recombinant protein in an aqueous solution at different concentrations. This is the first step of the study in order to comparatively evaluate the specific selected protein lower concentration and show unexpected recovery texture uniformity. It was evaluated without any other ingredient, only ADF-4 in aqueous solution. Figure 4 illustrates that the best performance was obtained with 0,01% of ADF-4 in water.

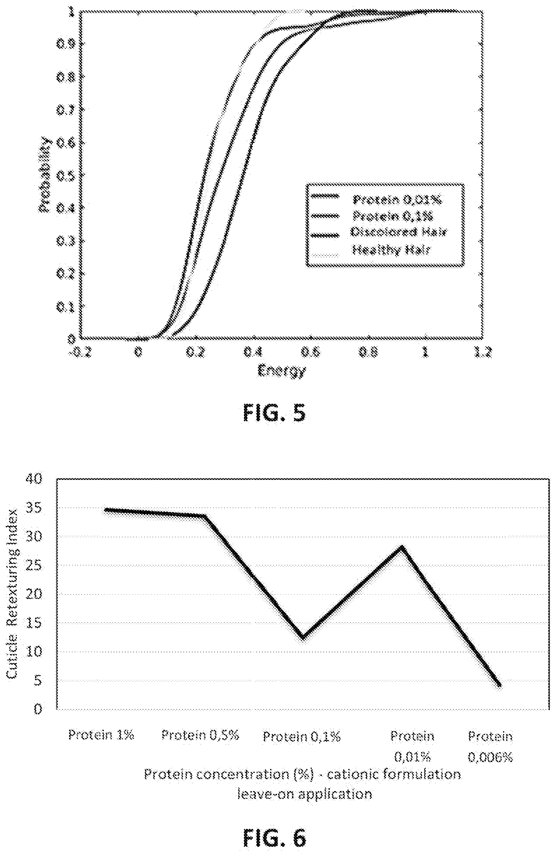

Figure 5 illustrates a graphic evaluating the re-texturization of the recombinant protein incorporated in a cationic formulation at different concentrations for a leave-on application. This figure illustrates that ADF-4 keeps the performance at 0.01% concentration even in the presence of other ingredients.
Figure 6 shows the recombinant protein response (recovery of texture uniformity) in a cationic formulation with leave-on application for different ADF-4 concentrations.

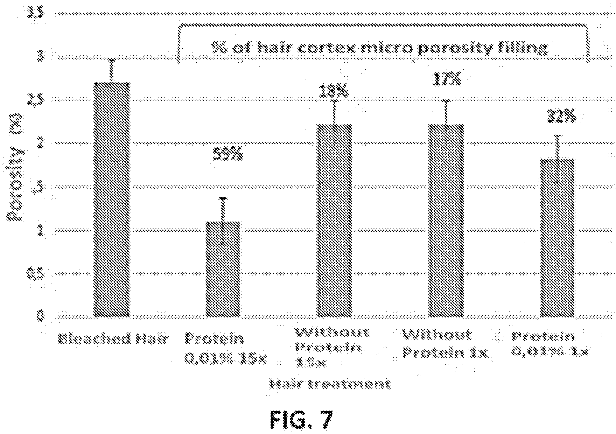

Figure 7 shows a graphic evaluation of the hair cortex porosity after one application (1x) or 15 applications (15x) of ADF-4 in a cationic formulation with rinse-off application.

### DISCLOSURE OF THE INVENTION

The present invention refers to the selection and use of the specific recombinant spider silk protein, called ADF-4 recombinant, in a specific amount, to produce a cosmetic composition providing unexpected effects, particularly desired deep and superficial treatment of keratin fibers.

Surprisingly, the present invention aims to utilize a recombinant protein derived from a spider silk recombinant protein as an active ingredient for cosmetic applications, particularly for hair treatment, focusing on anti-breakage, strength, pore filling, superficial re-texturing and damage repair.

Any mention to ADF-4 in the present specification means ADF-4 recombinant silk protein.

According to the invention, the ADF-4 recombinant is used in the selected range of 0.06 to 0.008%, preferably 0.01%, based on the total weight of the composition.

It was unexpectedly verified that the ADF-4 at the selected range according to the present invention does not act as a mere occlusive film as disclosed in the prior art, but it is able to provide superficial treatment of keratin fibers by recovering texture uniformity by filling porous channels in the hair cuticle and advantageously deep treatment of keratin fibers by filling in porous channels in the bulk cortex.

Therefore, the present invention refers, additionally, to a cosmetic composition comprising 0.06 to 0.008%, preferably 0.01% of ADF-4 recombinant protein, based on the total weight of the composition, and cosmetic acceptable vehicles.

The present invention has surprisingly determined that the efficacy response decreases with decreasing protein concentration. However, in a preferred embodiment of the invention, it has been surprisingly proven that at the specific concentration of 0.01% the ADF-4 recombinant protein provides excellent performance (Figure 6).

The present invention shows that the efficacy response decreases with decreasing protein concentration. However, surprisingly it has been proven that at the specific concentration of 0.01% excellent performance is obtained.

The cosmetic composition according to the present invention may be in the form of any hair product, such as shampoo, conditioner, mask, cream, emulsion, serum, gel, spray, ointment, dyes, leave-on, leave-in, rinse-off, etc.

A further aspect of the present invention is a method for treating keratin fibers which comprises applying the cosmetic composition defined in the present invention to the keratin fibers and optionally rinsing the keratin fibers.

By keratin fibers it is meant, without limitations, natural and synthetic fibers in general, such as hair, eyebrows, eyelashes, mustache, beard and other hair of the human or animal body, particularly damaged hair.

In accordance with the present invention for "superficial care" it is meant the performance in the keratinous barrier layer, through a film forming mechanism wherein the ADF-4 spider silk recombinant protein according to the present invention acts by adsorption in the most damaged regions of the hair shaft cuticle filling in porous channels in the cuticle structure and then retexturing the hair.

By retexturing and variations of this expression it is meant in the present invention the recovery texture uniformity.

By "deep care" it is meant the permeation effect through the barrier keratinous layer as far as the inner layers filling in porous channels in the bulk cortex, replenishing protein mass.

By "superficial and deep care" it is meant the simultaneous performance both in the cuticle and cortex.

Damaged hair is understood as broadly as possible, *i.e.* hair that has been influenced by external agents such as smoothing, heat treatment, discoloration, coloring, UV rays, among other.

As far as the Applicant knows, recombinant spider silk proteins do not fill the porous channels in the hair cuticle and, in general, have average molecular weight preventing any action in the cortex. Therefore, the proteins and compositions thereof known in the prior art do not provide superficial and deep care.

In a particular embodiment, the specific ADF-4 recombinant protein used in the specific selected amount according to the present invention, is dispersed in water in the form of a gel. Said dispersion comprises: (a) a water soluble fraction and (b) a water dispersed fraction forming a gel.

The water soluble fraction (a) that contains the recombinant protein in the form of hydrogel is believed to be able to diffuse into the cuticle; and the water dispersed fraction (b) that contains the protein in the form of protein aggregates is believed to be able to be adsorbed in the cortex. Therefore, simultaneous superficial and deep actions are surprisingly obtained, by retexturing hair fiber and filling in the channels in the cortex bulk.

As a result from retexturing and porosity benefits, the present invention improves elasticity and strength.

The following examples are merely illustrative of the present invention.

### EXAMPLES

Some tests were performed with the objective of evaluating the behavior of different compositions and concentrations of the ADF-4 recombinant protein (called as protein in the examples for easy reference) in the hair shaft:
- Methodology to evaluate hair surface texture - example 1;
- Test of ADF-4 alone in aqueous solution, with no other ingredients present - example 2 (test 2); and
- Test of ADF-4 incorporated into a cationic base to evaluate performance maintenance of 0.01% without interference from any other ingredient of a cationic hair formulation - example 2 (test 3).

### Preparation of the hair to be tested

In the tests that will be described below, particularly in the examples 2, 3 and 4, hair shaft samples were used, and, to perform those tests the pretreatment described below was conducted.

Hair strands of usual blond hair (control or healthy) were used. All strands were pre-washed with sodium lauryl ether sulfate (SLES) or Sodium lauryl sulfate (SLS) 5% (0.5 mL / strand), and rinsed with water for 1 min. Then, treatments for producing damages (oxidative damage) in the usual blond hair samples were conducted: The control strands were washed and discolored as recommended by the manufacturer of the product, *i.e.* in a non-metallic container the bleaching powder (Biocolor^{®} Rapid Bleach - Niasi^{®}) and hydrogen peroxide (20 vol) (Biocolor^{®} Creamy Oxygenated Water - Niasi^{™}) were added in the ratio 1:3 and mixing it for 2 minutes. 10 mL of the mixture was applied to each hair strand massaging for 5 minutes. After left to stand for 30 minutes at 36ºC the strands were rinsed off for 1 minute and the excess water removed.

The ingredients of Biocolor^{®} Rapid Bleach - Niasi^{®} are: sodium silicate, ammonium per sulfate, silica, magnesium trisilicate, cellulose gum, modified cornstarch, sorbitol, sodium lauryl sulfate, EDTA, fragrance, hydrolyzed collagen, keratin powder.

The active ingredient, representative of the present invention, was recombinant protein solutions in water, and cationic formulation at concentrations of 1%, 0.1%,0.01% and 0.006% of protein.

Hair treatment (control) was performed by wetting the strands (5g) for 20 seconds and removing excess of water. Then, 0.5 ml of 10% SLES was applied, massaging the strands for 60 seconds, followed by rinsing for 30 seconds.

For the strands that were treated with protein or with the cationic base, after this procedure 0.5 mL of the samples were applied to the strands (5 g) that were massaged for 60 seconds. When the process had rinse-off, these strands were rinsed for 30 seconds under running water. In the case of leave-on application there is no rinse.

In all procedures the strands are acclimatized for 24 hours at 20 °C before the tests are performed.

### Statistical evaluation

Additionally, statistical evaluation was used to calculate the results that will be described in the tests. The energy data obtained by analysis of the hair images through second-order statistics are non-parametric. The energy data obtained also reflects texture parameter, that is, the lower the energy the better the hair surface texture.

Therefore, the Kruskal-Wallis statistical test for K non-parametric samples was used.

The test was able to differentiate healthy hair from discolored hair and the effectiveness of different concentrations of protein.

### Example 1 - ANALYSIS OF THE ADF-4 PROTEIN

This example refers to an analysis of the ADF-4 silk recombinant protein in the form of hydrogel (Silkgel^{®}) by fluorescence analysis in excitation at 280 nm, by determination of protein diameter in solution, using DLS (Dynamic Light Scattering).

### Fluorescence Emission Results

Samples of recombinant protein were received at room temperature. For this analysis, the protein in the form of a powder, solubilized in 45% EtOH: acetate solution pH 4, concentration of 0.1 mg mL⁻¹ was used.

Although the recombinant protein does not contain tryptophan residues, it contains tyrosine residues, which fluoresce when they are excited at 280 nm. In fact, Figures 1a and 1b show that there is emission of fluorescence at this wavelength. Figure 1c shows the emission spectrum of a spider silk recombinant protein sample of the literature, and confirms the existence of the folding protein in the sample submitted for analysis.

### Circular Dichroism Results

Although, theoretically, the recombinant protein is mainly in the form of β-sheets, the CD spectrum obtained is not the characteristic of this secondary structure (Figure 2a). However, the literature shows that expression of amino acid repeat motifs, without repetition and a fusion between these motifs, yields completely different spectra (Figure 2b). The spectrum obtained experimentally resembled the recombinant protein spectra containing both repeating amino acid and non-repeating moieties.

### Dynamic light scattering (DLS) Results

This technique allows evaluating the diameter of the recombinant protein in solution. Again, the protein in the form of powder solubilized in 33% EtOH: acetate solution pH 4.0 and at a concentration of 0.1 mg mL⁻¹ was used.

Twenty (20) measures were taken. The equipment provides 1, 2 or 3 peaks in size, indicating that the recombinant protein might be forming aggregates. For calculation of a mean, measurements with lower polydispersity were used that contained a peak around 1 nm (recombinant protein size range). In an analogous way, the gel sample was analyzed, but with dissolution in water.

In this way, a value between 100 nm and 300nm of hydrodynamic diameter of the silk recombinant protein in the form of hydrogel is reached. Therefore the results for DLS analyzes indicated the diameter for gel sample from 100nm to 300nm.

It should be noted that signals were generated relative to the aggregates of the recombinant protein (greater than 10 nm and greater than 100 nm). The gel sample only showed signs related to recombinant protein aggregates, which leads to the conclusion that the 3% of recombinant protein into gel formulation is not completely water soluble, generating stable suspensions of proteinaceous aggregates resistant to high temperatures.

This corroborates the results of the protein's effectiveness in filling the micropores of the hair fiber cortex for soluble and dispersed protein's phases.

### Conclusion

Fluorescence emission spectra and circular dichroism meet the results found in the literature. In addition, by means of light scattering techniques, the presence of nanometric suspensions of this sample is confirmed.

### Example 2 - EVALUATION OF EFFICACY ON THE SURFACE OF THE HAIR SHAFT (CUTICLE) - HAIR RETEXTURE.

In this example the surface of the hair shaft was evaluated by analysis of digital images of texture by second order statistics.

The lower the energy read, the better the surface texture (repair) of the hair cuticle (Figures 4 and 5).

### Test 1: Differentiation between healthy control hair and of discolored hair

A comparison between discolored hair, healthy control hair and the qualitative evaluation of protein's efficacy for recovery surface hair texture uniformity is shown in Figure 3.

### Test 2 - Evaluation of the recombinant protein in aqueous solution in different concentrations in the "Retexturing" of discolored hairs.

In this test the following samples and concentrations were evaluated:
- Control hair (healthy);
- discolored hair (damaged)
- discolored hair treated with protein 1% in water
- discolored hair treated with protein 0.1% in water
- discolored hair treated with protein 0.01% in water

The aqueous solution was applied just one time.

A significant difference in texture after protein application at different concentrations in discolored hair is observed. It is also seen that concentrations of 0.1% and 0.01% return the discolored hair to healthy hair condition (Figure 4).

It was found that the lower the protein concentration the greater the efficacy.

### Test 3 - Evaluation of the recombinant protein in cationic formulation in different concentrations in the "retexturing" of discolored hairs:

In this test the following samples and concentrations were evaluated:
- control hair(healthy);
- discolored hair (damaged);
- discolored hair treated with protein 0.1% leave-on application; and
- discolored hair treated with protein 0.01% leave-on application.

It was found that 0.01% concentration shows the greater efficacy.

The main components of the tested cationic compositions are illustrated below in Table 1:

| Function | Class of ingredients | Minimum (%) | Maximum (%) |
|---|---|---|---|
| Cationic surfactant | Quaternary ammonium salt compounds or fatty amine salts | 1 | 5 |
| Co- Surfactant | Fatty alcohol | 2 | 6 |
| adjuvants | Organic acids, organic basic, preservative, | 0.01 | 5 |
| Active ingredients | Sr_spider polypeptide ADF-4 | 0.01 | 1.0 |
| Inert excipients /Vehicle | Water | qsp | qsp |

There is a significant difference in texture after application of the recombinant protein in different concentrations in discolored hair (Figure 5).

At concentrations of 0.1% in the cationic base, the hair condition is close to the damage hair.

It is also seen that at concentrations of 0.01% in the cationic base, the hair returns discolored to the healthy hair condition.

In other words, it is obtained a superior effect in a lower concentration.

### Conclusions over tests:

The re-texturization tests were performed in order to measure the surface hair texture homogeneity obtained by analysis of the hair. The smaller the energy measured better is the texture of the hair surface. In the re-texturization tests, it was observed that:
- All the recombinant protein concentrations showed efficacy in relation to discolored hair, through the reduction of the energy, *i.e.* of the damage to the surface as measured.
- The effective concentration of recombinant protein (or less energy) for re-texturization is:
   - In water, protein 0.01% yielded the lowest energy - test 2,
   - in cationic formulation, protein 0.01% yielded the lowest energy - test 3,

### Example 3 - MICRO-TOMOGRAPHY OF X-RAYS FOR HAIRS - EVALUATION OF POROSITY (CORTEX)

Refers to a technique for the evaluation of micro-damage (porosity of the cortex) of the hair fiber in X-ray microtomography. Protein study was performed using ADF-4 incorporated in a cationic formulation (table 1) and in rinse off application. It was also performed multiple application (15x).

The test was carried out for hair evaluation of damaged hair strand with oxidative damaged (discoloration).

It was evaluated the effect of a placebo (cationic formulation without protein) and cationic formulation with 0.01% of protein. For both samples, it was evaluated the effect for 1 rinse-off application and 15 rinse-off application.

The compositions tested are illustrated in the table 1 described above.

### Preparation of hair samples with application of the active recombinant protein in damaged hair:

Hair treatment (control) was performed by wetting the strands (5g) for 20 seconds and removing excess of water. Then, 0.5 ml of 10% SLES was applied, massaging the strands for 60 seconds, followed by rinsing for 30 seconds.

For the strands that were treated with protein or with the cationic base, after this procedure 0.5 mL of the samples were applied to the strands (5 g) that were massaged for 60 seconds. When the process had rinse-off, these strands were rinsed for 30 seconds under running water. In the case of leave-on composition there is no rinse.

In all procedures the strands are acclimatized for 24 hours at 20°C before the tests are performed.
**Application ratio 1:1:** In 0.5 g of hair strands it was applied 0.5 g of solution with protein.
**Application of rinse off:** The strands were massaged with fingers to spread the recombinant protein solution along the length of the hair shaft and the hair was allowed to dry at room temperature for 24h. After application of the active, the strands were dried in the environment for 24 hours in laboratory at temperature around 25 °C. The hair fibers were moun ted on glass slides and send to analyses.

The steps for evaluation of micro-damage (porosity of the cortex) are:
- Preparation of hair samples in an acclimatized condition of the assembly room and equipment. Temp: 21 to 23ºC and (relative humidity) U.R.: 45-60%.
- Acquisition and processing conditions;
- Binarization and separation of hair strands; and
- Determination of porosity of hair strands analyzed

The results are illustrated in Figure 7.

### Conclusions:

The reduction of the porosity of discolored hair when using protein in a hair conditioning base was 59% in multiple applications compared to placebo (base without the protein) which was 18% under the same conditions (Figure 7).

Tests above show unexpected results obtained by the non-obvious selected specific narrow range (about 0.06 to about 0.008%), mainly by the preferred amount (about 0.01% )of ADF-4 recombinant protein based on the total weight of the composition, independent on the cosmetic acceptable vehicles. Cosmetic compositions containing such a selected specific amount of the ADF-4 recombinant protein effectively provide double effect, i.e. superficial treatment of keratin fibers by recovering texture uniformity by filling porous channels in the hair cuticle and advantageous deep treatment of keratin fibers by filling in porous channels in the bulk cortex.

## Claims

1. A COSMETIC COMPOSITION FOR TREATING KERATIN FIBERS comprising 0.06 to 0.008% of ADF-4 recombinant silk protein based on the total weight of the composition, and cosmetic acceptable vehicles.

2. THE COSMETIC COMPOSITION of claim 1 comprising 0.01% of ADF-4 recombinant silk protein based on the total weight of the composition, and cosmetic acceptable vehicles.

3. THE COSMETIC COMPOSITION of claim 1 wherein the ADF-4 recombinant silk protein is dispersed in water in the form of a gel.

4. THE COSMETIC COMPOSITION of claim 1 wherein the ADF-4 recombinant silk protein is dispersed in water in the form of a gel comprising (a) a water soluble fraction that contains the recombinant protein in the form of hydrogel and (b) a water dispersed fraction that contains the protein in the form of protein aggregates forming a gel.

5. THE COSMETIC COMPOSITION of claim 1 wherein is a shampoo, conditioner, mask, cream, emulsion, serum, gel, spray, ointment, dye, leave-on, leave-in or rinse-off composition.

6. A METHOD FOR TREATING KERATIN FIBERS comprising to apply the cosmetic composition defined in one of claims 1-5 to the keratin fibers and optional rinse.

7. THE METHOD of claim 6 wherein the keratin fibers are natural and synthetic fibers.

8. THE METHOD of claim 6 wherein the fibers are hair, eyebrows, eyelashes, mustache, beard and other hair of the human or animal body, particularly damaged hair.

9. USE OF ADF-4 RECOMBINANT SILK PROTEIN to prepare a cosmetic composition, wherein said recombinant protein is used in an amount of 0.06 to 0.008% based on the total weight of a final cosmetic composition.

10. THE USE of claim 9 wherein said recombinant protein is used in an amount of 0.01% based on the total weight of a final cosmetic composition.

11. THE USE of claim 9, wherein the ADF-4 recombinant silk protein is dispersed in water in the form of a gel.

12. THE USE of claim 9, wherein the ADF-4 recombinant silk protein is dispersed in water in the form of a gel comprising (a) a water soluble fraction that contains the recombinant protein in the form of hydrogel and (b) a water dispersed fraction that contains the protein in the form of protein aggregates forming a gel.

## Patentansprüche

1. KOSMETISCHE ZUSAMMENSETZUNG ZUR BEHANDLUNG VON KERATINFASERN, umfassend 0,06 bis 0,008 % rekombinantes Seidenprotein ADF-4, bezogen auf das Gesamtgewicht der Zusammensetzung, und kosmetisch akzeptable Trägerstoffe.

2. KOSMETISCHE ZUSAMMENSETZUNG nach Anspruch 1, umfassend 0,01 % rekombinantes Seidenprotein ADF-4, bezogen auf das Gesamtgewicht der Zusammensetzung, und kosmetisch akzeptable Trägerstoffe.

3. KOSMETISCHE ZUSAMMENSETZUNG nach Anspruch 1, wobei das rekombinante Seidenprotein ADF-4 in Wasser in Form eines Gels dispergiert ist.

4. KOSMETISCHE ZUSAMMENSETZUNG nach Anspruch 1, wobei das rekombinante Seidenprotein ADF-4 in Wasser in Form eines Gels dispergiert ist, das (a) eine wasserlösliche Fraktion, die das rekombinante Protein in Form eines Hydrogels enthält, und (b) eine wasserdispergierte Fraktion, die das Protein in Form von Proteinaggregaten enthält, die ein Gel bilden, umfasst.

5. KOSMETISCHE ZUSAMMENSETZUNG nach Anspruch 1, wobei es sich um ein Shampoo, eine Spülung, eine Maske, eine Creme, eine Emulsion, ein Serum, ein Gel, ein Spray, eine Salbe, ein Färbemittel, eine Leave-on- oder Leave-in- oder ausspülbare Zusammensetzung handelt.

6. VERFAHREN ZUR BEHANDLUNG VON KERATINFASERN, umfassend das Auftragen der in einem der Ansprüche 1-5 definierten kosmetischen Zusammensetzung auf die Keratinfasern und optional das Ausspülen.

7. VERFAHREN nach Anspruch 6, wobei die Keratinfasern natürliche und synthetische Fasern sind.

8. VERFAHREN nach Anspruch 6, wobei es sich bei den Fasern um Haare, Augenbrauen, Wimpern, Schnurrbart, Bart und sonstige Haare des menschlichen oder tierischen Körpers, insbesondere geschädigtes Haar, handelt.

9. VERWENDUNG VON ADF-4-REKOMBINANTEM SEIDENPROTEIN zur Herstellung einer kosmetischen Zusammensetzung, wobei das rekombinante Protein in einer Menge von 0,06 bis 0,008 %, bezogen auf das Gesamtgewicht der endgültigen kosmetischen Zusammensetzung, verwendet wird.

10. VERWENDUNG nach Anspruch 9, wobei das rekombinante Protein in einer Menge von 0,01 %, bezogen auf das Gesamtgewicht der endgültigen kosmetischen Zusammensetzung, verwendet wird.

11. VERWENDUNG nach Anspruch 9, wobei das rekombinante Seidenprotein ADF-4 in Wasser in Form eines Gels dispergiert ist.

12. VERWENDUNG nach Anspruch 9, wobei das rekombinante Seidenprotein ADF-4 in Wasser in Form eines Gels dispergiert ist, das (a) eine wasserlösliche Fraktion, die das rekombinante Protein in Form eines Hydrogels enthält, und (b) eine wasserdispergierte Fraktion, die das Protein in Form von Proteinaggregaten enthält, die ein Gel bilden, umfasst.

## Revendications

1. COMPOSITION COSMÉTIQUE DESTINÉE À TRAITER DES FIBRES DE KÉRATINE comprenant 0,06 à 0,008 % de protéine de soie recombinante ADF-4 sur la base du poids total de la composition, et véhicules acceptables cosmétiques.

2. COMPOSITION COSMÉTIQUE selon la revendication 1 comprenant 0,01 % de la protéine de soie recombinante ADF-4 sur la base du poids total de la composition, et véhicules acceptables cosmétiques.

3. COMPOSITION COSMÉTIQUE selon la revendication 1 dans laquelle la protéine de soie recombinante ADF-4 est dispersée dans l'eau sous la forme d'un gel.

4. COMPOSITION COSMÉTIQUE selon la revendication 1 dans laquelle la protéine de soie recombinante ADF-4 est dispersée dans l'eau sous la forme d'un gel comprenant (a) une fraction soluble dans l'eau qui contient la protéine recombinante sous la forme d'un hydrogel et (b) une fraction dispersée dans l'eau qui contient la protéine sous la forme d'agrégats de protéines formant un gel.

5. COMPOSITION COSMÉTIQUE selon la revendication 1 dans laquelle il s'agit d'un shampoing, d'un après-shampoing, d'un masque, d'une crème, d'une émulsion, d'un sérum, d'un gel, d'un spray, d'une pommade, d'un colorant, d'une composition sans rinçage ou à rincer.

6. PROCÉDÉ DESTINÉ À TRAITER DES FIBRES DE KÉRATINE comprenant l'application de la composition cosmétique définie dans l'une des revendications 1 à 5 sur les fibres de kératine et un rinçage facultatif.

7. PROCÉDÉ selon la revendication 6 dans lequel les fibres de kératine sont des fibres naturelles et synthétiques.

8. PROCÉDÉ selon la revendication 6 dans lequel les fibres sont les cheveux, les sourcils, les cils, la moustache, la barbe et d'autres poils du corps humain ou animal, en particulier des poils abîmés.

9. UTILISATION DE LA PROTÉINE DE SOIE RECOMBINANTE ADF-4 pour préparer une composition cosmétique, dans laquelle ladite protéine recombinante est utilisée en une quantité de 0,06 à 0,008 % sur la base du poids total d'une composition cosmétique finale.

10. UTILISATION selon la revendication 9, dans laquelle ladite protéine recombinante est utilisée en une quantité de 0,01 % sur la base du poids total d'une composition cosmétique finale.

11. UTILISATION selon la revendication 9, dans laquelle la protéine de soie recombinante ADF-4 est dispersée dans l'eau sous la forme d'un gel.

12. UTILISATION selon la revendication 9, dans laquelle la protéine de soie recombinante ADF-4 est dispersée dans l'eau sous la forme d'un gel comprenant (a) une fraction soluble dans l'eau qui contient la protéine recombinante sous la forme d'un hydrogel et (b) une fraction dispersée dans l'eau qui contient la protéine sous la forme d'agrégats de protéines formant un gel.
